# EUROPEAN PATENT APPLICATION

(11) **EP 4 759 357 A1**
(43) Date of publication of application: **17.06.2026**
(21) Application number: 25220718.8
(22) Date of filing: 04.12.2025
(51) Int. Cl.: A61N 5/10

(54) **ENERGY TREATMENT PLAN ADJUSTMENT METHOD AND APPARATUS**

(30) Priority: 12.12.2024 US 202418978700
(71) Applicant: Siemens Healthineers International AG, 6312 Steinhausen (CH)
(72) Inventor: HARHANEN, Lauri Oskari, 00270 Helsinki (FI); WAREING, Todd, Blackfoot, 83221 (US); HARJU, Ari, 02140 Espoo (FI); SABEL, Martin, 6332 Hagendorn (CH); KAUPPINEN, Juha, 02770 Espoo (FI)
(74) Representative: Mathisen & Macara LLP

(57) **Abstract**

A control circuit can be configured to access both an energy treatment plan 201 for a particular patient using a particular energy treatment apparatus, which energy treatment plan was optimized with respect to at least one clinical goal, and dose information 202 indicating that the energy treatment plan includes at least one area in at least one patient volume where an administered dose will not comport with the at least one clinical goal. The control circuit can then perform 203 adjoint transport analysis for the at least one patient volume to characterize an effect of therapeutic energy on the at least one clinical goal and adjust 204 the energy treatment plan in the at least one area as a function of the characterized effect of therapeutic energy on the at least one clinical goal.

## Description

### TECHNICAL FIELD

These teachings relate generally to treating a patient's planning target volume with energy pursuant to an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-apparatus parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Plan optimizers sometimes use dose calculation algorithms that have lower accuracy than final dose calculation algorithms in order to enable optimization in an acceptable time frame. In some cases, a final dose calculation may reveal that the plan does not fully meet the requisite clinical goals notwithstanding that the optimizer's dose calculator indicates acceptable plan quality. Examples in these regards can include so-called hot spots in non-targeted patient volumes and/or so-called cold spots in a targeted patient volume.

### SUMMARY

In one aspect, the present invention provides a method as defined in claim 1. Optional features are specified in the dependent claims.

In another aspect, the present invention provides apparatus as defined in claim 7. Optional features are specified in the dependent claims.

### BRIEF DESCRIPTION OF DRAWINGS

The above needs are at least partially met through provision of the energy treatment plan adjustment method and apparatus described in the following detailed description, particularly when studied in conjunction with the drawings, wherein:
FIG. 1 comprises a block diagram as configured in accordance with various embodiments of these teachings; and
FIG. 2 comprises a flow diagram as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, pursuant to these various embodiments, a control circuit can access both an energy treatment plan for a particular patient using a particular energy treatment apparatus, which energy treatment plan was optimized with respect to at least one clinical goal as well as dose information indicating that the energy treatment plan includes at least one area in at least one patient volume where an administered dose will not comport with the at least one clinical goal. The control circuit can then perform adjoint transport analysis for the at least one patient volume to characterize an effect of therapeutic energy on the at least one clinical goal followed by adjusting the energy treatment plan in the at least one area as a function of the characterized effect of therapeutic energy on the at least one clinical goal.

By one approach, the aforementioned at least one area can comprise an area (such as, for example, at least one of a hot spot and a cold spot) within at least one of a patient target volume (such as a tumor) and a non-targeted volume (such as an organ-at-risk).

By one approach, performing the adjoint transport analysis for the at least one patient volume to characterize an effect of therapeutic energy on the at least one clinical goal can comprise characterizing an individual effect on dosing of each of a plurality of energy beamlets.

By one approach, adjusting the energy treatment plan in the at least one area as a function of the characterized effect of therapeutic energy on the at least one clinical goal can comprise adjusting at least one physical parameter of the particular energy treatment apparatus. The at least one physical parameter of the particular energy treatment apparatus can comprise, as one example, a planned position of at least one multi-leaf collimator leaf.

By one approach, adjusting the energy treatment plan in the at least one area as a function of the characterized effect of therapeutic energy on the at least one clinical goal can comprise presenting information regarding the characterized effect of therapeutic energy on a user interface to a user to facilitate the user making changes to the energy treatment plan.

These teachings are highly flexible in practice and will accommodate any of a variety of modifications and/or supplemental features. As one example in these regards, these teachings will further accommodate modifying the energy treatment plan as a function of the aforementioned adjustment of the energy dosing to provide a modified energy treatment plan. As another example, and in such a case, these teachings will further accommodate optionally administering therapeutic energy to the particular patient using the particular energy treatment apparatus using the modified energy treatment plan.

So configured, these teachings will accommodate, for example, using an adjoint formulation of a transport equation to calculate how incoming radiation affects the dose in an area where the dose from the final calculation does not meet clinical goals. Where prior approaches might require thousands or even millions of computations to reach a similar result, these teachings will accommodate using only one calculation for each distinct area. In a typical application setting, the calculation results proffered by these teachings are similar in accuracy to the results garnered with conventional dose calculations.

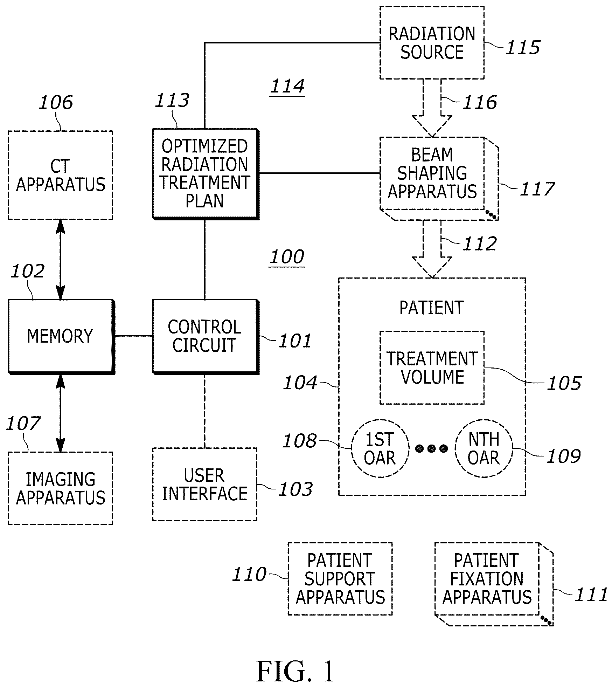

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware apparatus (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware apparatus (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated apparatus or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory apparatuses that collectively comprise the "memory" of this apparatus 100.

In addition to information such as optimization information for a particular patient and information regarding a particular radiation treatment apparatus as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both nonvolatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless apparatuses, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-apparatus parameters during each of a plurality of sequential exposure fields. In this case the energy-based treatment plan is generated through an optimization process, examples of which are provided further herein.

By one approach the control circuit 101 can operably couple to an energy-based treatment apparatus 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment apparatus/apparatuses. In a typical application setting the energy-based treatment apparatus 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment apparatus 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

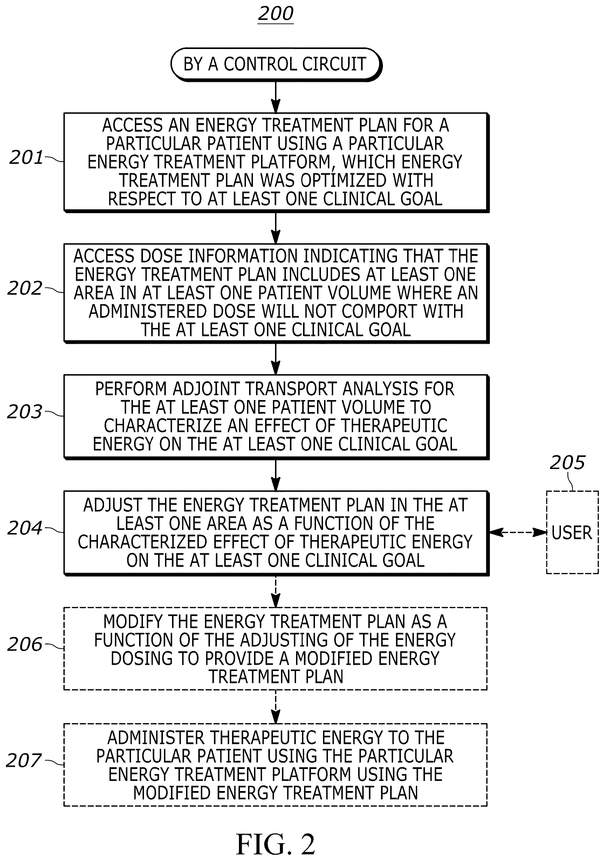

Referring now to FIG. 2, a process 200 that can be carried out, for example, in conjunction with the above-described application setting (and more particularly via the aforementioned control circuit 101) will be described. Generally speaking, this process 200 serves to facilitate adjusting an optimized radiation treatment plan 113 to thereby facilitate treating a particular patient with therapeutic radiation using a particular radiation treatment apparatus per that optimized radiation treatment plan.

At block 201, this process 200 provides for accessing an energy treatment plan for a particular patient using a particular energy treatment apparatus, which energy treatment plan was optimized with respect to at least one clinical goal (such as a goal specifying a radiation dosage level to be attained (or avoided) or to be otherwise observed in a particular patient volume). Various approaches to such optimization are known in the art. As the present teachings are not particularly sensitive to any particular choices in these regards, further elaboration regarding optimization is not provided here.

At block 202, the control circuit 101 accesses dose information (for example, by accessing the aforementioned memory 102) indicating that the energy treatment plan includes at least one area in at least one patient volume (such as a patient's target volume and/or a non-targeted volume) where an administered dose will not comport with the at least one clinical goal. That area may not comport with the at least one clinical goal by reason of being, for example, a hot spot (for example, in a non-targeted patient volume) or a cold spot (for example, in a targeted patient volume).

At block 203, the control circuit 101 performs adjoint transport analysis for the at least one patient volume to characterize an effect of therapeutic energy on the at least one clinical goal. The latter may comprise, by one approach, characterizing an individual effect on dosing of each of a plurality of energy beamlets.

Adjoint transport analysis is a computational method to efficiently calculate system responses to a multitude of perturbations. This technique involves solving the adjoint equation, which is mathematically derived from an original (forward) transport equation, effectively reversing the direction of information propagation. The solution to the adjoint equation provides a sensitivity profile or importance function that, when integrated with a source term or perturbation, yields the response of the system to that perturbation. These teachings leverage this approach to compute a response to many different sources or changes in boundary conditions with a single adjoint solution, as opposed to solving the forward problem multiple times for each perturbation, thus significantly reducing computational effort and time.

Beamlets in the context of radiation treatment of a tumor refer to the small, individual rays of radiation that are part of a larger, more complex radiation beam used in advanced radiation therapy techniques. These beamlets can be individually modulated in intensity and directed with high precision to conform to the three-dimensional shape of patient volumes, allowing, for example, for the delivery of a high dose of radiation to malignant cells while minimizing exposure and potential damage to surrounding healthy tissue. The ability to control the intensity and direction of each beamlet can serve to enhance the effectiveness of energy-based therapy and improve patient outcomes. Beamlets are a typical part of planning intensity-modulated radiation therapy (IMRT) and volumetric modulated arc therapy (VMAT).

At block 204, the control circuit 101 can adjust the energy treatment plan in the aforementioned at least one area as a function of the characterized effect of therapeutic energy on the at least one clinical goal. By one approach, adjusting the energy treatment plan can comprise adjusting at least one physical parameter of the particular energy treatment apparatus, such as, but not limited to, a planned position of at least one multi-leaf collimator leaf. (Multi-leaf collimators are comprised of a plurality of individual parts (known as "leaves") that are formed of a high atomic numbered material (such as tungsten) that can move independently in and out of the path of the radiation-therapy beam in order to selectively block (and hence shape) the beam. Typically the leaves of a multi-leaf collimator are organized in pairs that are aligned collinearly with respect to one another and which can selectively move towards and away from one another. A typical multi-leaf collimator has many such pairs of leaves, often upwards of twenty, fifty, or even one hundred such pairs.)

By one approach, these teachings will accommodate presenting information regarding the characterized effect of the therapeutic energy on a display (such as the aforementioned user interface 103) to a user 205 to facilitate the user 205 making one or more change to the energy treatment plan as described above.

By one optional approach, and referring to optional block 206, the control circuit 101 can then modify the energy treatment plan as a function of the aforementioned adjustment of the energy dosing to provide a modified energy treatment plan. At optional block 207, these teachings will then accommodate, if desired, administering therapeutic energy to the particular patient using the particular energy treatment apparatus using the aforementioned modified energy treatment plan.

Further details that comport with these teachings will now be presented. It will be understood that these specific details are intended to serve an illustrative purpose and are not intended to suggest any particular limitations with respect to these teachings.

An energy treatment plan can be thought of as consisting of thousands or millions of tiny beamlets. The adjoint formulation can be used to characterize the individual effect of each of those beamlets to the dose in the area where dose does not match clinical goals (either by exceeding the goal, or falling short in those regards). Evaluating the effect of individual beamlets to a small area can be several orders of magnitude slower using conventional radiation transport techniques in comparison to adjoint transport. In particular, existing solutions calculate the dose from each beamlet separately, thereby resulting in thousands or even millions of dose calculations. In contrast, the adjoint approach espoused by these teachings switches the viewpoint and calculates how incoming radiation affects a particular area. As a beneficial consequence, only one calculation is needed for each distinct area. In addition, the results of that one calculation can be similar in accuracy to the results of conventional dose calculations.

In intensity modulated radiation therapy, this information can be used directly to determine an update to the plan. By one approach, and combined with an additional dose calculation for the plan update, a user can be presented with an interactive tool to choose a compromise between different goals when there is an apparent conflict. In volumetric modulated arc therapy, these teachings can serve to identify specific leaves of a multi-leaf collimator whose position(s) affect the dose in the unacceptable area (and targeted modifications in those regards can then be determined). Also, these teachings can be used to calculate updates to plans that decrease the differences in structure average doses between optimizer and final dose calculations.

Further aspects of these teachings are provided by the subject matter of the following clauses (where it will be understood that any of these clauses can be combined with any one of more of the other clauses as appropriate).

Clause 1. A method comprising: by a control circuit: accessing an energy treatment plan for a particular patient using a particular energy treatment apparatus, which energy treatment plan was optimized with respect to at least one clinical goal; accessing dose information indicating that the energy treatment plan includes at least one area in at least one patient volume where an administered dose will not comport with the at least one clinical goal; performing adjoint transport analysis for the at least one patient volume to characterize an effect of therapeutic energy on the at least one clinical goal; adjusting the energy treatment plan in the at least one area as a function of the characterized effect of therapeutic energy on the at least one clinical goal.

Clause 2. The method of clause 1 wherein the at least one area comprises an area within at least one of a patient target volume and a non-targeted volume.

Clause 3. The method of clause 2 wherein the at least one area comprises at least one of a hot spot and a cold spot.

Clause 4. The method of any of clauses 1 through 3 wherein performing adjoint transport analysis for the at least one patient volume to characterize an effect of therapeutic energy on the at least one clinical goal comprises characterizing an individual effect on dosing of each of a plurality of energy beamlets.

Clause 5. The method of any of clauses 1 through 4 wherein adjusting the energy treatment plan in the at least one area as a function of the characterized effect of therapeutic energy on the at least one clinical goal comprises adjusting at least one physical parameter of the particular energy treatment apparatus.

Clause 6. The method of clause 5 wherein the at least one physical parameter of the particular energy treatment apparatus comprises a planned position of at least one multi-leaf collimator leaf.

Clause 7. The method of any of clauses 1 through 6 wherein adjusting the energy treatment plan in the at least one area as a function of the characterized effect of therapeutic energy on the at least one clinical goal comprises presenting information regarding the characterized effect of therapeutic energy on a user interface to a user to facilitate the user making changes to the energy treatment plan.

Clause 8. The method of any of clauses 1 through 7 further comprising:
modifying the energy treatment plan as a function of the adjusting of the energy dosing to provide a modified energy treatment plan.

Clause 9. The method of clause 8 further comprising: administering therapeutic energy to the particular patient using the particular energy treatment apparatus using the modified energy treatment plan.

Clause 10. An apparatus comprising: a control circuit configured to: access an energy treatment plan for a particular patient using a particular energy treatment apparatus, which energy treatment plan was optimized with respect to at least one clinical goal; access dose information indicating that the energy treatment plan includes at least one area in at least one patient volume where an administered dose will not comport with the at least one clinical goal; perform adjoint transport analysis for the at least one patient volume to characterize an effect of therapeutic energy on the at least one clinical goal; adjust the energy treatment plan in the at least one area as a function of the characterized effect of therapeutic energy on the at least one clinical goal.

Clause 11. The apparatus of clause 10 wherein the at least one area comprises
an area within at least one of a patient target volume and a non-targeted volume.

Clause 12. The apparatus of clause 11 wherein the at least one area comprises at least one of a hot spot and a cold spot.

Clause 13. The apparatus of any of clauses 11 through 12 wherein the control circuit is configured to perform adjoint transport analysis for the at least one patient volume to characterize an effect of therapeutic energy on the at least one clinical goal by characterizing an individual effect on dosing of each of a plurality of energy beamlets.

Clause 14. The apparatus of any of clauses 11 through 13 wherein the control circuit is configured to adjust the energy treatment plan in the at least one area as a function of the characterized effect of therapeutic energy on the at least one clinical goal by adjusting at least one physical parameter of the particular energy treatment apparatus.

Clause 15. The apparatus of clause 14 wherein the at least one physical parameter of the particular energy treatment apparatus comprises a planned position of at least one multi-leaf collimator leaf.

Clause 16. The apparatus of any of clauses 11 through 15 wherein the control circuit is configured to adjust the energy treatment in the at least one area as a function of the characterized effect of therapeutic energy on the at least one clinical goal by presenting information regarding the characterized effect of the therapeutic energy on a user interface to a user to facilitate the user making changes to the energy treatment plan.

Clause 17. The apparatus of any of clauses 11 through 16 wherein the control circuit is further configured to: modify the energy treatment plan as a function of the adjusting of the energy dosing to provide a modified energy treatment plan.

Clause 18. The apparatus of clause 17 wherein the control circuit is further configured to: administer therapeutic energy to the particular patient using the particular energy treatment apparatus using the modified energy treatment plan.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method comprising:
by a control circuit:
accessing an energy treatment plan for a particular patient using a particular energy treatment apparatus, which energy treatment plan was optimized with respect to at least one clinical goal;
accessing dose information indicating that the energy treatment plan includes at least one area in at least one patient volume where an administered dose will not comport with the at least one clinical goal;
performing adjoint transport analysis for the at least one patient volume to characterize an effect of therapeutic energy on the at least one clinical goal;
adjusting the energy treatment plan in the at least one area as a function of the characterized effect of therapeutic energy on the at least one clinical goal.

2. The method of claim 1 wherein performing adjoint transport analysis for the at least one patient volume to characterize an effect of therapeutic energy on the at least one clinical goal comprises characterizing an individual effect on dosing of each of a plurality of energy beamlets.

3. The method of claim 1 or 2 wherein adjusting the energy treatment plan in the at least one area as a function of the characterized effect of therapeutic energy on the at least one clinical goal comprises adjusting at least one physical parameter of the particular energy treatment apparatus.

4. The method of claim 3 wherein the at least one physical parameter of the particular energy treatment apparatus comprises a planned position of at least one multi-leaf collimator leaf.

5. The method of any one of claims 1 to 4 wherein adjusting the energy treatment plan in the at least one area as a function of the characterized effect of therapeutic energy on the at least one clinical goal comprises presenting information regarding the characterized effect of therapeutic energy on a user interface to a user to facilitate the user making changes to the energy treatment plan.

6. The method of any one of claims 1 to 5 further comprising:
modifying the energy treatment plan as a function of the adjusting of the energy dosing to provide a modified energy treatment plan.

7. An apparatus comprising:
a control circuit configured to:
access an energy treatment plan for a particular patient using a particular energy treatment apparatus, which energy treatment plan was optimized with respect to at least one clinical goal;
access dose information indicating that the energy treatment plan includes at least one area in at least one patient volume where an administered dose will not comport with the at least one clinical goal;
perform adjoint transport analysis for the at least one patient volume to characterize an effect of therapeutic energy on the at least one clinical goal;
adjust the energy treatment plan in the at least one area as a function of the characterized effect of therapeutic energy on the at least one clinical goal.

8. The method of any one of claims 1 to 6, or the apparatus of claim 7, wherein the at least one area comprises an area within at least one of a patient target volume and a non-targeted volume.

9. The method of any one of claims 1 to 6 and 8, or the apparatus of claim 7 or 8, wherein the at least one area comprises at least one of a hot spot and a cold spot.

10. The apparatus of claim 7, 8 or 9 wherein the control circuit is configured to perform adjoint transport analysis for the at least one patient volume to characterize an effect of therapeutic energy on the at least one clinical goal by characterizing an individual effect on dosing of each of a plurality of energy beamlets.

11. The apparatus of any one of claims 7 to 10 wherein the control circuit is configured to adjust the energy treatment plan in the at least one area as a function of the characterized effect of therapeutic energy on the at least one clinical goal by adjusting at least one physical parameter of the particular energy treatment apparatus.

12. The apparatus of claim 11 wherein the at least one physical parameter of the particular energy treatment apparatus comprises a planned position of at least one multi-leaf collimator leaf.

13. The apparatus of any one of claims 7 to 12 wherein the control circuit is configured to adjust the energy treatment in the at least one area as a function of the characterized effect of therapeutic energy on the at least one clinical goal by presenting information regarding the characterized effect of the therapeutic energy on a user interface to a user to facilitate the user making changes to the energy treatment plan.

14. The apparatus of any one of claims 7 to 13 wherein the control circuit is further configured to:
modify the energy treatment plan as a function of the adjusting of the energy dosing to provide a modified energy treatment plan.

15. The apparatus of claim 14 wherein the control circuit is further configured to:
administer therapeutic energy to the particular patient using the particular energy treatment apparatus using the modified energy treatment plan.
